(19) Europäisches Patentamt
European Patent Office
Office européen des brevets

(11) Publication number: 0 298 755 A1

# (12) EUROPEAN PATENT APPLICATION

(21) Application number: 88306243.2

(22) Date of filing: 08.07.88

(51) Int. Cl.[4]: C 07 K 17/00
G 01 N 33/74, G 01 N 33/535,
C 12 N 9/00

(30) Priority: 08.07.87 US 71063

(43) Date of publication of application:
11.01.89 Bulletin 89/02

(84) Designated Contracting States:
AT BE CH DE ES FR GB GR IT LI LU NL SE

(71) Applicant: TECHNICON INSTRUMENTS CORPORATION
511 Benedict Avenue
Tarrytown, New York 10591 (US)

(72) Inventor: Hwang, Deng R.
52 Tarry Hill Road
Tarrytown, New York 10591 (US)

Scott, Mary E.
7374 Brookside Parkway
Middleburg, Ohio 44130 (US)

Hedaya, Eddie
500-402 Higpoint Drive
Hartsdale, New York 10530 (US)

(74) Representative: Wain, Christopher Paul et al
Northumberland House 303-306 High Holborn
London WC1V 7LE (GB)

## (54) Protein conjugates and their preparation.

(57) Protein conjugate compounds useful in liposome immunoassays have the formula:

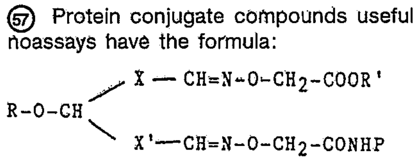

I

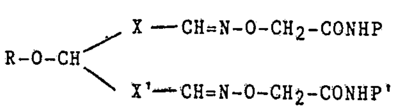

II

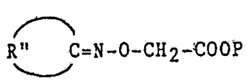

III

wherein R is derived from an organic compound containing 1,2-dihydroxy groups which groups are subject to oxidative cleavage; X and X′ are the side chain moieties connecting the R moiety to the carbon atoms resulting from oxidative cleavage of the 1,2-dihydroxy groups; P and P′ are proteins or protein derivatives; and R′ is H or ethyl; and R″ is derived from a steroid having at least one carbonyl group which forms the oximino moiety =N-O.

EP 0 298 755 A1

**Description**

## PROTEIN CONJUGATES AND THEIR PREPARATION

This invention relates to novel immunoreactive protein conjugates useful for homogeneous liposome immunoassays. More particularly, this invention provides a rapid and efficient approach to conjugates such as digoxin-protein conjugates, which are useful for liposome immunoassay.

Digoxin is a potent cardiac glycoside. Toxic amounts of digoxin exert undesirable and potentially lethal electrophysiological effects (Hoffman et al, The Pharmacological Basis of Therapeutics, Gilman, 6th ed., p 729, N.Y. 1980). Accordingly, various immunoassay methods for cardiac glycosides are now widely used clinically as aids in the determination of appropriate dosage schedules for patients receiving these drugs. Because digoxin is too small a molecule to be antigenic by itself, it is necessary to conjugate digoxin covalently as a hapten to antigenic carriers, for example, human serum albumin (HSA), bovine serum albumin (BSA) or keyhole limpet hemocyanin (KLH) in order to elicit digoxin-specific antibodies in experimental animals for use in immunoassay. The preparation of immunoreactive digoxin derivatives is typically carried out by the procedure of Butler et al (Proc. Natl. Acad. Sci., U.S.A., 1967, 57, 71-78; and Methods in Enzymology, Academic Press, 84, 558-577 (1982)) which is based on the work of Erlanger and Beiser, Proc. Natl. Acad. Sci., 52, 68 (1964). The reaction sequence involved periodate cleavage of the terminal sugar ring (digitoxose or rhamnose) followed by reaction with a protein carrier, enzyme or related biological molecule, and finally reductive amination with sodium borohydride. Thus, digoxin-HSA (Butler et al supra), dogoxin-BSA (Smith et al, Biochemistry, 9, 331-337 (1970)), mellitin-ouabain (Freytag et al, J.. of Immunological Methods, 70, 113-140 (1984)), and digoxin-dibenzo-18-crown-6- (Keating et al, Anal. Chem. 56, 801-806 (1984)) conjugates have been prepared using the aforementioned reaction sequence.

U.S. Patent 4,115,539 discloses a method of preparing digoxin conjugates using isocyanates based upon tyrosine methyl ester. U.S. Patent 4,297,273 and 4,363,759 disclose ways to prepare chemiluminescent phythalaldehyde-labeled digoxigenin. United States Patent No. 4,029,880 describes methods for preparing radioisotopically labeled derivatives of digoxin and digitoxin. U.S. Patent 4,342,826 employs the procedure of Butler et al supra to prepare a non-characterized digoxin-phosphatidylethanolamine conjugate. This preparation was a very complex mixture having low conjugate purity.

We have now discovered certain new conjugates (including digoxin conjugates) which are useful for sensitive liposome immunoassays.

According to the invention, there is provided a protein conjugate compound of the formula:

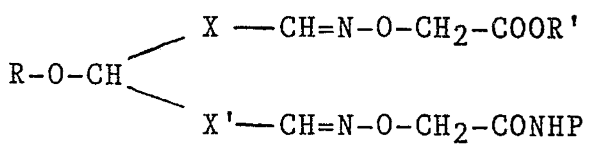

I

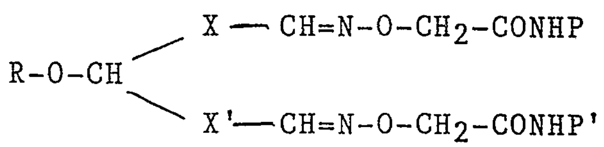

II

wherein R is derived from an organic compound containing 1,2-dihydroxy groups which groups are subject to oxidative cleavage; X and X′ are the side chain moieties connecting the carbon atoms resulting from oxidative cleavage of the 1,2-dihydroxy groups with the R moiety; P and P′ are proteins or protein derivatives; and R′ is ethyl or H.

In one embodiment of the invention, digoxin-protein conjugates are prepared that are useful for both heterogeneous and homogeneous immunoassays, such as sensitive, immunoreactive and stable digoxin assay tracers. Efficiency of formation of separated immunocomplex enzyme activity is greater with this conjugate than with commercially available conjugates. Furthermore, the assays are reproducible and efficient. A typical digoxin assay for which compounds of the invention are useful is one using magnetic separation.

In an embodiment, R is derived from a cardiac glycoside such as digoxin, digitoxin, gitoxin, oubain, digitonin and the like, and P and P′ are proteins. For compounds of Formula I, R′ is preferably H and P is protein, such as an antibody or an enzyme, more preferably an enzyme. Proteins such as alkaline phosphatase, horse radish

peroxidase, beta-galactosidase, human serum albumin, bovine serum albumin and immunoglobulins (including the antibody and its fragments) are preferred proteins. A preferred immunoglobulin is immunoglobulin G. For compounds of Formula II, P and P′ are proteins as described above.

In another embodiment of this invention, compounds are included having the formula

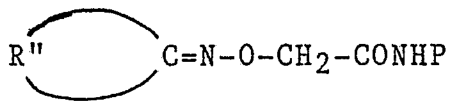

III

wherein R″ is derived from a steroid having at least one carbonyl group, said carbonyl group forming said oximino moiety =N-O-, and P is a protein as described above. Illustrative steroids include testosterone, 6-keto-estradiol, and digoxin.

The invention also includes a method of preparing a protein conjugate having formula I or formula II or the formula III, comprising the steps of

(a) oxidatively cleaving the 1,2-dihydroxy groups of an organic compound;

(b) reacting the resulting 1,2-dialdehyde groups with carboxy methoxylamine to form the corresponding dioxime; and

(c) reacting protein with said dioxime intermediate.

A liposomal immunoassay such as that disclosed in U.S. patent specification 4,342,826 or 4,235,792 to which reference should be made, which uses the herein disclosed compounds, is also within the scope of this invention.

A synthetic sequence for preparing the protein conjugates of this invention is illustrated using digoxin and phospholipid (instead of protein), in Scheme A provided hereinafter.

Scheme A

Synthesis Of Digoxin-DPPE Conjugates

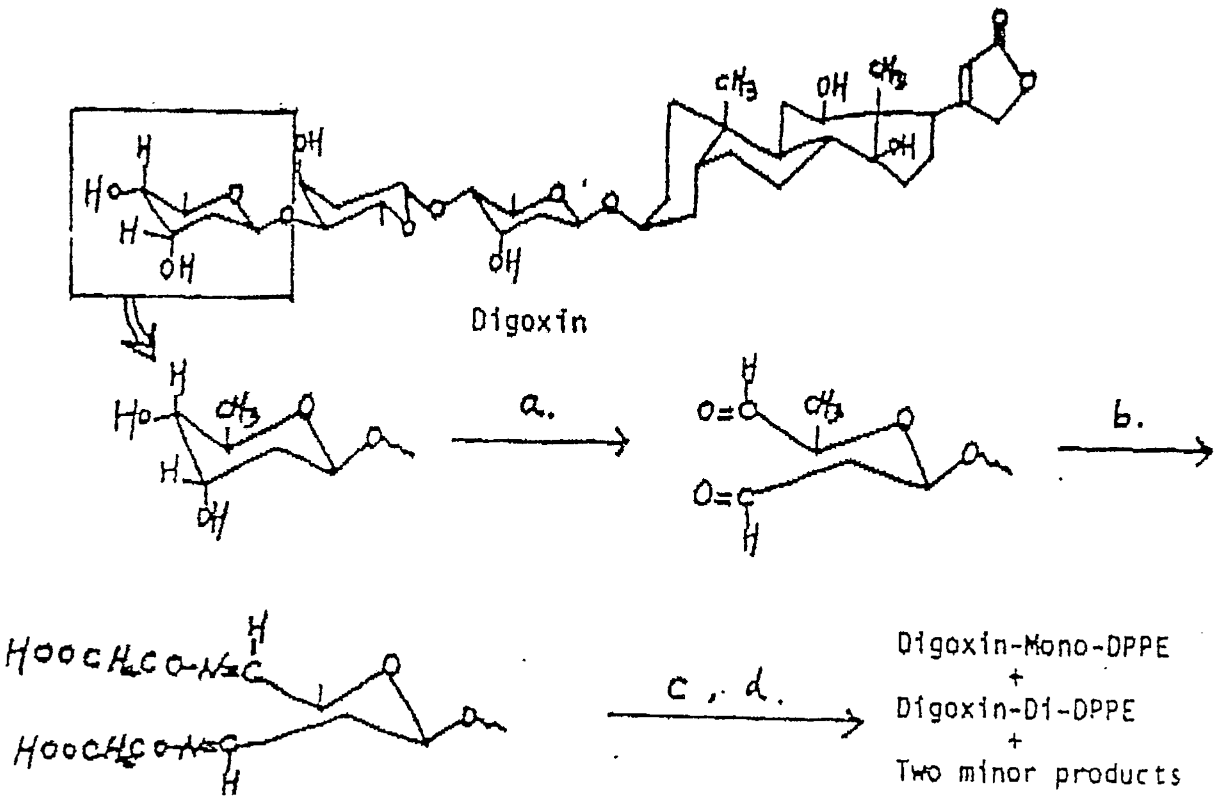


a, sodium periodate

b, $(NH_2OCH_2COOH)_2.HCl/NaOAc + EtOH$

c, NHS + D.C.C. ⟶ active ester

d, dipalmitoyl phosphatidyl ethanolamine/$CHCl_3/Et_3N$

The terminal digitoxose in digoxin is cleaved to give dialdehyde in quantitative yield by using, e.g., sodium periodate under nitrogen atmosphere. Thin layer chromatography (silica gel, Merck) with the solvent system chloroform/methanol (10/1 by volume) shows one homogeneous spot at $R_f$ 0.16 (detected by spraying with methanol/concentrated sulfuric acid (9/1 by volume) and warming briefly to develop a dark brown color. The condensation reaction of digoxin dialdehyde and carboxy methoxyamine hemihydrochloride proceeds rapidly in sodium acetate/ethanol under nitrogen atmosphere. Quantitative yields of di- (O-carboxymethyl) oxime (TLC: $R_f$ 0.04-0.12 solvent system chloroform/methanol 6/1 by volume) were obtained. The digoxin derivative was used immediately in the next reaction step. The carboxy functionalities of the dioxime are then reacted with N-hydroxysuccinimide in the presence of dicyclohexylcarbodiimide to give an active ester. The dioxime active ester is then condensed with dipalmitoyl phosphatidylethanolamine (DPPE) with gentle heating for seventy-two hours. The reaction was monitored closely by TLC. Thin layer chromatography with the solvent system chloroform/methanol/water (75/25/3 by volume) showed two major components at $R_f$ 0.20 and 0.13 along with N-hydroxysuccinimide at at $R_f$ 0.30. The phospholipid moiety of the conjugates were detected by molybdate blue spray. The excess N-hydroxysuccinimide was removed from the reaction mixture by preparative LC (Kieselgel, 200g, glass column (2.5 x 50 cm), solvent system chloroform/methanol/water (2/8/1 by volume)). Pure digoxin-di-DPPE conjugate (0.1348g, 20%; $R_f$ 0.30) and digoxin-mono DPPE conjugate (0.1424g, 26.5%; $R_f$ 0.15) were isolated from crude reaction mixture by preparative TLC in chloroform/methanol/water (2/8/1 by volume). Two other minor products were also isolated. The structures of the mino products were not identified.

The structure proof of the two major conjugates was extracted from the IR, UV, high resolution proton NMR spectra, and fact atom bombardment (FAB) mass spectra.

The advantages of the above-described synthetic procedure reside in its ability and facility to efficiently provide a relatively stable, storable, yet activatable, oxime intermediate. This intermediate overcomes the disadvantages inherent in the Butler et al procedure discussed earlier. These include the propensity for the dialdehyde intermediate to undergo deleterious side-reactions, particularly in the presence of amine derivatives of lesser reactivities such as phospholipids. An additional advantage is that the claimed procedure provides products which can be readily isolated, characterized and purified in contrast to Butler, which to our knowledge, yields a sufficiently complex mixture thwarting the desired product characterization.

The synthetic method described herein is also applicable to the preparation of analogous phospholipid conjugates involving linkage through a sugar ring such as digitoxin, gitoxin, ouabain, digitonin and related cardiac glycosides, or those involving steroids which can be modified, to form oxime derivatives remote from key functionalities important for immunorecognition by specific antibodies.

## EXAMPLE I

### Synthesis of Digoxin DPPE Conjugates

#### A) Digoxin Dialdehyde

Digoxin (0.4985 g, 0.64 mmole) is dissolved in 10 ml of chloroform/methanol (3/1.5) and placed into a 100 ml two-necked flask. Sodium periodate (0.3102 g, 1.4 mmoles) is dissolved in 4 ml distilled water and placed into a pressure equalized addition funnel. The periodate solution is slowly added to the flask while stirring and under nitrogen. A white precipitate is immediately formed and the reaction is complete within 15 minutes after addition of the periodate. Reaction progress is monitored by TLC (E.M. Merck, pre-coated TLC sheets, silica gel 60 F254 0.2 mm thickness) in chloroform/methanol 10/1 by volume ($R_f$ 0.16 = dialdehyde, one homogeneous spot; $R_f$ 0.07 - digoxin. Both spots became dark brownish when sprayed the TCL plate with methanol/concentrated sulfuric acid (9/1 by volume) and placed in 100°C oven). The reaction mixture is evaporated on a rotary evaporator and brought up in 30 ml of chloroform and 3 ml of water. The cloudy solution is extracted and the aqueous layer washed three times with 10 ml chloroform. The organic phases are combined (60 ml) and dried over magnesium sulfate. The organic solvents are evaporated to dryness. A light yellow brownish oily material is left. This material is used immediately in the next reaction.

#### B) Digoxin Di-(O-carboxymethyl) Oxime

Carboxy methoxylamine hemihydrochloride (0.3119 g, 1.4 mmoles) and sodium acetate (0.2260 g, 1.6 mmoles) are dissolved in 3 ml water and placed into a 50 ml 2-necked flask. The digoxin dialdehyde, dissolved in 1.3 ml ethanol, is placed into a pressure equalized funnel and slowly added to the flask while stirring and under nitrogen. The reaction is complete within ten minutes (TLC: chloroform/methanol 6/1 by volume, $R_f$ 0.09-0.13). The reaction mixture is evaporated to dryness and dissolved in 20 ml ethyl acetate and 3 ml water. The organic layer is separated and the aqueous layer is washed three times with 5.0 ml ethyl acetate. The organic layers are combined and dried over anhydrous magnesium sulfate. The solution is filtered and

evaporated to dryness. The residue is dried for 30 minutes under high vacuum (0.1 mm Hg) and used immediately for the next step.

C) Preparation of N-Hydroxy-succinimide Ester of Digoxin Di (O-Carboxymethyl) Oxime

Dicyclohexylcarbodiimide (DCC 0.2805 g, 1.3 mmoles) is dissolved in 6 ml dry DMF and placed into a 50 ml 2-necked flask. The solution is cooled in an ice-water bath (4°C). Digoxin di(O-carboxymethyl) oxime, dissolved in 80 ml DMF, is slowly added while stirring and under nitrogen to the flask. Immediately afterwards, N-hydroxy-succinimide solution (0.1500 g, 1.3 mmoles, in 6 ml DMF) is likewise added. Reaction progress is monitored to TLC [chloroform/methanol/water 75/25/3 by volume, $R_f$ 1.0 (DCC), 0.75 (dioxime NHS active ester), 0.34 (NHS), 0.1 (dioxime). The reaction is continued at 4°C under nitrogen for 18 hours.

The desired product possesses the following characteristics in TLC: (1) homogeneous UV detectable spot (short wavelength), (2) the homogeneous spot turns brownish when spraying the TLC plate with methanol/concentrated sulfuric acid (9/1 by volume) and warming the plate briefly in 100°C oven.

The reaction mixture is filtered to remove dicyclohexyl urea and the crude reaction mixture is used for the next step.

D) Preparation of Digoxin-DPPE Conjugates

The crude dioxime active ester (17 ml reaction mixture) is placed into a 100 ml 2-necked flask. A suspension of DPPE (0.4431 g, 0.64 mmoles, dispersed in 30 ml dry chloroform and 0.7 ml of triethyl amine) is placed into an addition funnel and slowly added to the flask while stirring under nitrogen and being protected from light. The mixture was heated gently (40-50°C) and continued for 72 hours. The reaction was monitored by TLC (solvent system chloroform/methanol/water 75/25/3 by volume; $R_f$ 0.75 (active ester), 0.52 (unknown 1), 0.45 (unknown 2), 0.30 (NHS), 0.21 DPPE, 0.20 (disubstituted conjugate), 0.13 (monosubstituted conjugate). The reaction mixture is complex and the above are the major identified products. The phospholipid moiety of the conjugates was detected by molybdate blue spray. The reaction mixture was evaporated and brought up in 10 ml chloroform/methanol/water (2/8/1). The N-hydroxy-succinimide was removed from the mixture by LPLC [(Kieselgel 200 g, glass column (2.5 cm x 50 cm), solvent system chloroform/methanol/water (2/8/1 by volume)]. Pure mono and disubstituted conjugates can be obtained by preparative TLC in chloroform/methanol/water (2/8/1 by volume, $R_f$ 0.30 (disubstituted); 0.15 (monosubstituted). Pure digoxin-di-DPPE conjugate (0.1349 g 20%) and digoxin-mono-DPPE conjugate (0.1424 g 26.5%) were obtained. Two other minor products were also isolated. The structures of the minor products was not identified.

E) NMR, UV, IR and FAB Data

The NMR, UV and IR and fast atom bombardment (FAB) mass spectra data are summarized in Charts 1 and 2 provided hereinbelow:

CHART 1

Digoxin-Mono-DPPE

NMR (300 MHz, $CDCl_3$)
0.8 (singlet, 3H, $18CH_3$), 0.96 (singlet, 3H, 19 CH,)
0.90 (triplet, 6H, terminal methyl group in phospholipid)
2.23-1.05 [ (complex multiplet, 83H, 2 $(CH_2)_{12}$, 3 CH (digitoxose ring), 3 $CH_2$ (digitoxose ring), 8 CH, (digoxigenin ring), 2 $\underline{CH_2}$ $(CH_2)_{12}$]
2.32 (two overlapping triplets, 4H, 2 $CH_2CO$)
4.72-3.05 [ (complex multiplet, 27H, $CH_2OCOR$, 9 CH (digitoxose ring proton), 6 CH (digoxigenin ring proton), glycero $CH_2$-O-P protons, ethanolamine $CH_2$-O-P, -$OCH_2$-CO- and -$CH_2NH_2$)]
4.95 (multiplet, 5H, $CH_2$ in lactone and 3 protons at $C_1$, $C_1$ , and $C_1$ in digitoxose)
5.25 (multiplet, 1H, -CH-OCOR)
5.95 (singlet, 1H, lactone, -C=CH)
UV (Cary 219, $CHCl_3$) max, 241 nm (ε 1744)
IR (KBr, Perkin Elmer 1430 ratio reading, $cm^{-1}$): 3435 (broad OH), 2923, 2852, 1743 (ester), 1668, 1622.

CHART 2

Digoxin-Di-DPPE

NMR (300MHz, $CDCL_3$)
0.82 (singlet, 3H, $18CH_3$), 0.94 (singlet, 3H, 19 $CH_3$)
0.90 (triplet, 12H, terminal methyl group in phospholipid)
2.32 (multiplet, 8H, 4 $CH_2$-CO)
2.2-1.05 (complex multiplet)
4.75-2.8 (complex multiplet)
4.9 (multiplet, 5H, $CH_2$ in lactone and 3 protons at $C_1$, $C_1$, and $C_1$ in digitoxose)
5.25 (multiplet, 2H, 2 CH-OCOR)
5.95 (singlet, 1H, Lactone -C=CH)
UV (Cary 219, $CHCl_3$) max. 241 nm ($\varepsilon$ 2071)
IR (KBr, Perkin Elmer 1430 ratio reading, $cm^{-1}$) : 3427 (broad OH) 2923, 2853, 1781, 1743, 1668.

As a further confirmation of the structure, positive ion fast atom bombardment (FAB) mass spectra ($^m/Z$) from purified conjugates in a thioglycerol matrix were obtained using the MS-50 high resolution mass spectrometer. The most intense peak appeared in the molecular ion region, representin $^m/Z$ of (M + metal) and the isotopically enriched species. The molecular ion for digoxin-mono-DPPE is 1621 (M + Na) and the molecular ion for digoxin-di-DPPE is 2311 (M + K).

F) Liposomal Immunoassay

Both conjugates are used in the liposomal immunoassay methods described in U.S. 4,342,826 (e.g. Example X) and in U.S. 4,235,792 with acceptable results.

EXAMPLE II

The procedure of Example I is repeated wherein, in lieu of digoxin, approximate stoichiometric equivalent amounts of the following compounds are used with comparable results:
digitoxin
gitoxin
ouabain
digitonin

EXAMPLE III

The procedure of Example I is repeated wherein, in lieu of dipalmitoyl phosphatidyl ethanolamine, an approximate stoichiometric equivalent amount of dimyristyl phosphatidyl ethanolamine is used to yield the corresponding product.

EXAMPLE IV

The procedure of Example I [(steps B), C) and D)] is repeated except that testosterone is used, in an approximate stoichiometric amount, in lieu of digoxin dialdehyde to obtain the corresponding conjugated phospholipid product.

A synthesis scheme similar to scheme A represents a synthetic sequence for preparing protein conjugates of this invention. Here, protein replaces DPPE at part "d",

Digoxin-Protein Conjugates

N-hydroxysuccinimide (NHS) active ester of digoxin di(O-carboxymethyl) oxime was prepared in large quantity. This ester is stable when stored under anhydrous condition and at -20 C. Because organic solvents such as DMF, DMSO, THF, and dioxane exert detrimental effects of the bioactivity of enzymes, the digoxin-protein conjugates are prepared under strenuous conditions. Choice of buffers, pH, amount of organic solvents used, initial molar ratio of activated digoxin and enzyme and purification procedures are important factors for the preparation of a conjugate with optimum enzymatic and immunoreactivity. The present process is applicable to a wide variety of biopolymers containing primary amino functionality. Specifically, the biopolymers of interest are alkaline phosphatase, beta-galactosidase, horse radish

peroxidase, human and bovine serum albumin and immunoglobulins and fractions thereof. These immunoglobulins may function as antibodies. A preferred immunoglobulin is immunoglobulin G. Conjugation, purification procedures and methods for characterization of a digoxin-protein conjugate are provided below.

The synthetic method described herein is also applicable to the preparation of analogous protein conjugates involving linkage through a sugar ring such as digitoxin, gitoxin and related cardiac glycosides, or those involving steroids which can be modified, to form oxime derivatives remote from key functionalities important for immunorecognition by specific antibodies.

## EXAMPLE V

### Synthesis of Digoxin-Alkaline Phosphatase Conjugate

An 8 mg/ml stock solution of digoxin N-hydroxysuccinimide active ester in DMF was prepared. 3.2ml of phosphate buffer (pH 7.6, 50 mM sodium phosphate + 1 mM magnesium chloride + 0.1 mM zinc chloride) was place in a 25 ml round bottom flask, and to this flask was added 20 mg of alkaline phosphatase (ALP). The buffer and alkaline phosphatase were stirred together at room temperature for 10 minutes. 0.250 ml of digoxin active ester was added dropwise while stirring. Stirring was at room temperature for 30 minutes and then at 4° C for 16 hours. This procedure resulted in conjugation of alkaline phosphatase to digoxin.

### Purification

After digoxin-alkaline phosphatase conjugation, the formed conjugate was subjected to chromatograph on a Sephadex® G-25 medium (2.2 cm diameter x 40 cm height) in Tris buffer. 30 drops of eluent of each fraction were collected. The fractions were pooled based on absorbance at 280 nm. Ten fractions had absorbance higher than 0.05. A small amount was set aside for protein determination. An appropriate amount of bovine serum albumin was added to the solution pool to form a concentration of 1 mg bovine serum albumin per ml solution. The conjugate was stored in an amber vial at 4°C.

### Conjugate Characterization

#### A. Protein Determination

Six standard solutions were prepared (0, 5, 10, 15, 20 and 35 ng/ml). 75 ul of a conjugate sample was diluted in 2,925 ul of a buffer.

0.8 ml of a standard or unknown was pipetted into a test tube, and 0.2 ml filtered dye (Bio-Rad protein assay dye reagent) concentrate was added to the same tube and mixed thoroughly. Approximately 5 minutes to one hour after addition, absorbance at 595 nm was read and compared with the zero standard. Each known standard solution was run two times, and each unknown solution was run three times.

The mean absorbance reading was plotted against the standard concentration. Unknown values were read from the linear portion of the standard curve and corrected for the dilution factor.

#### B. Enzyme Activity

Enzyme activity of the conjugate was determined by using a Technicon RA-1000® Random Access Clinical Analyzer. RA-1000® protocol was used for determining alkaline phosphate activity. The total enzyme activity was expressed as u/ml. The specific activity expressed as u/mg was found by dividing enzyme activity by protein concentration.

#### C. Hapten/Protein Ratio

The molar ratio of hapten and protein reflects the degree of incorporation of hapten onto enzyme. The molar rat of digoxin-alkaline phosphatase conjugates ranged from 0.1 to 5.0. The optimum ratio was determined from digoxin concentration (obtained by pepsin digestion and RIA method) a protein concentration.

Loss of activity during incubation at 37 C for various Digoxin: ALP ratios is shown in Graph I. Graph II shows specific activity and reaction ratio for various Digoxin: ALP ratios.

Immunoreactivity of Enzyme Labeled Digoxin

The immunoreactive performance of enzyme labeled digoxin tracer was evaluated in a laboratory immunoassay system at Technicon. 25 ul digoxin in clinical sample or in standard sample was incubated with 50 ul primary monoclonal antibody and 50 ul of a second antibody coupled to a magnetic particle for 15 minutes at 37° C. 50 ul of alkaline phosphatase labeled digoxin was then added to the reaction mixture. That mixture was then incubated for an additional 15 minutes at 37° C. The bound fraction was washed three times with a wash solution, and the bound and free fractions of tracer were thereafter separated by the second antibody coupled to the magnetic component. 250 ul of a substrate, p-nitrophenyl phosphate, was then added. The amount of enzyme tracer bound by magnetic component was inversely related to the concentration of digoxin in the sample. p-nitrophenol, the hydrolytic product of the substrate by bound enzyme, was measured at 405 nm.

Data below compares effect of antibody concentration of digoxin range for the tracer system of the present invention and a commercially used tracer system.

The antibody titration curve below (Graph III) the present invention with a representative commercial tracer.

EXAMPLE VI

Synthesis of Digoxin-Beta Galactosidase Conjugate

A synthesis procedure similar to the one used in Example V is used except alkaline phosphatase is substituted with beta-galactosidase, and the substrate p-nitrophenyl phosphate was substituted with O-nitrophenyl betagalactosidase.

**Claims**

1. A protein conjugate compound of the formula:

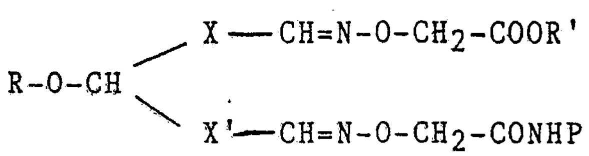

I

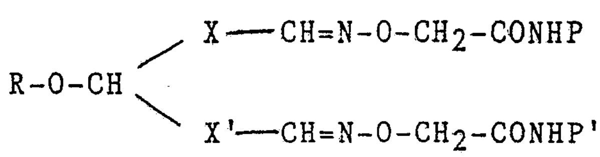

II

wherein R is derived from an organic compound containing 1,2-dihydroxy groups which groups are subject to oxidative cleavage; X and X′ are the side chain moieties connecting the R moiety to the carbon atoms resulting from oxidative cleavage of the 1,2-dihydroxy groups; P and P′ are proteins or protein derivatives; and R′ is H or ethyl.

2. A compound according to claim 1, which is of formula I or II wherein R is derived from digoxin, X is

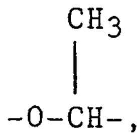

X′ is $-CH_2-$ and R′ (when present) is H.

3. A compound according to claim 1 or 2, wherein the protein is an enzyme or enzyme derivative.

4. A compound according to claim 3, wherein the enzyme is alkaline phosphatase, horseradish peroxidase, or beta-galactosidase.

5. A compound according to claim 1 or 2, wherein the protein is selected from bovine serum albumin or derivatives thereof, human serum albumin or derivatives thereof, an antibody or derivatives thereof, and immunoglobulines or derivatives thereof.

6. A compound according to claim 5, wherein the immunoglobulin is immunoglobulin G.

7. A compound according to claim 1 or 2, wherein the protein is a fraction of an immunoglobulin or a derivative thereof.

8. A protein conjugate compound having the formula

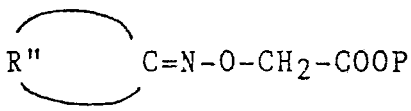

wherein R″ is derived from a steroid having at least one carbonyl group, said carbonyl group forming said oximino moiety =N-O, and P is a protein or protein derivative.

9. A compound according to claim 8, wherein the steroid is testosterone or 6-keto-estradiol.

10. A compound according to claim 8 or 9, wherein the steroid contains two or more carbonyl groups, at least one of which forms the oximino moiety to which the protein or protein derivative is attached.

11. A compound according to claim 1, wherein the group R-O- designates a steroid which contains a 1,2-dihydroxy group, which group is subject to oxidative cleavage.

12. A method of preparing a protein conjugate having formula I or formula II or the formula of claim 8, comprising the steps of

(a) oxidatively cleaving the 1,2-dihydroxy groups of an organic compound;

(b) reacting the resulting 1,2-dialdehyde groups with carboxy methoxylamine to form the corresponding dioxime; and

(c) reacting protein with said dioxime intermediate.

13. A method according to claim 12, wherein either compounds of formula I are prepared, or compounds of formula II are prepared, or a mixture containing compounds of formulae I and II is prepared.

14. A method according to claim 12 or 13 wherein there is prepared a conjugate as claimed in any of the claims 2 to 7, 9, 10 or 11.

15. A liposomal immunoassay for the determination of appropriate dosage schedules or cardiac glycosides and steroids for human administration which employs a protein conjugate as claimed in any of claims 1 to 11.

0298755

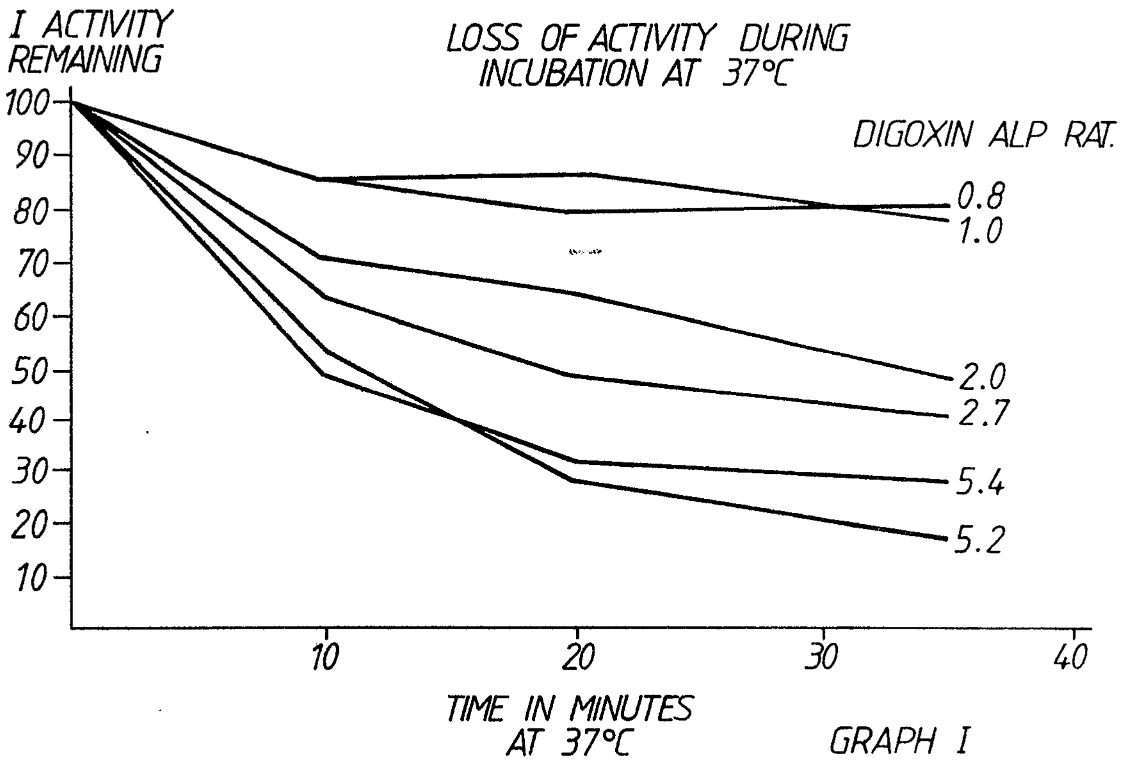


GRAPH I

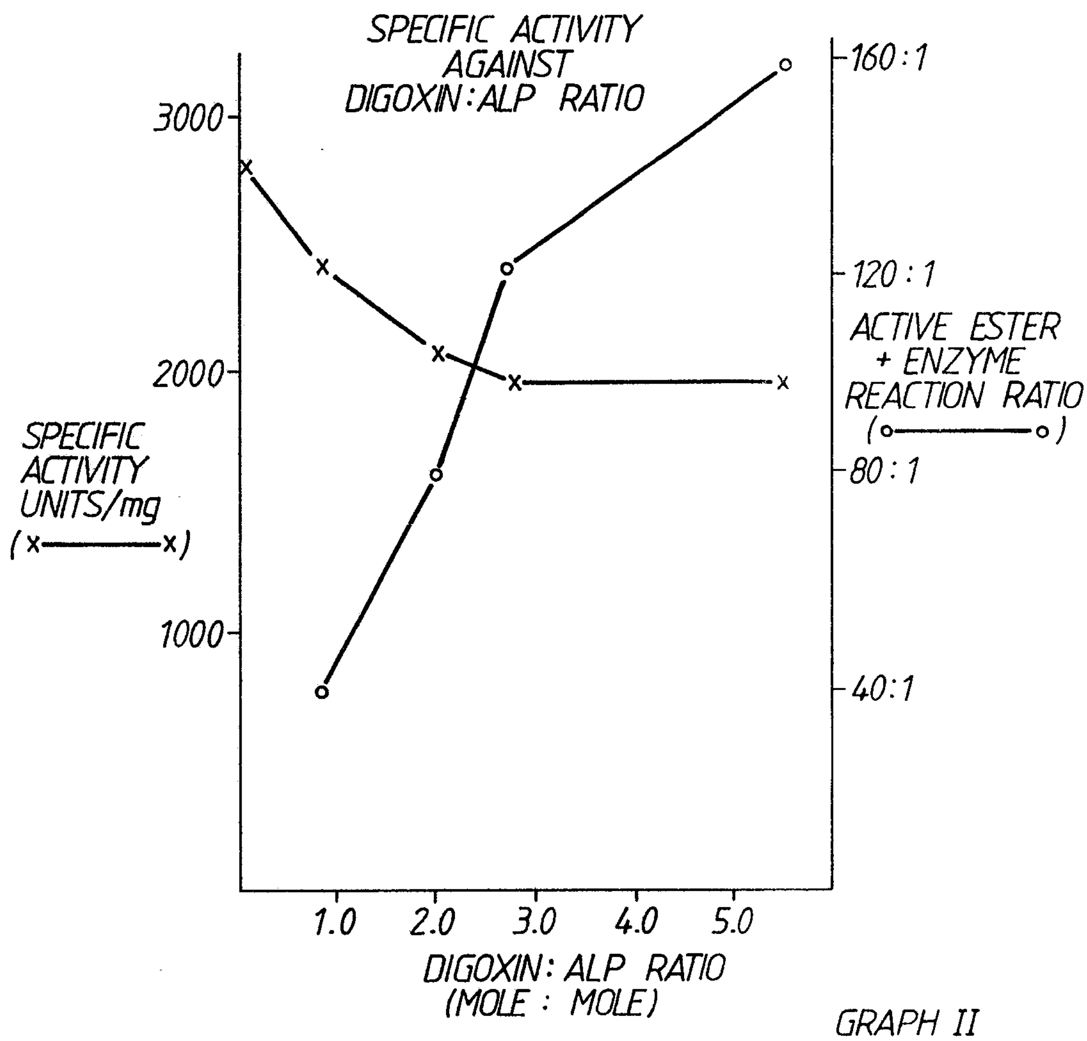


GRAPH II

13.10.88

0298755

EFFECT OF ab CONC. (AS5) ON DIGOXIN RANGE

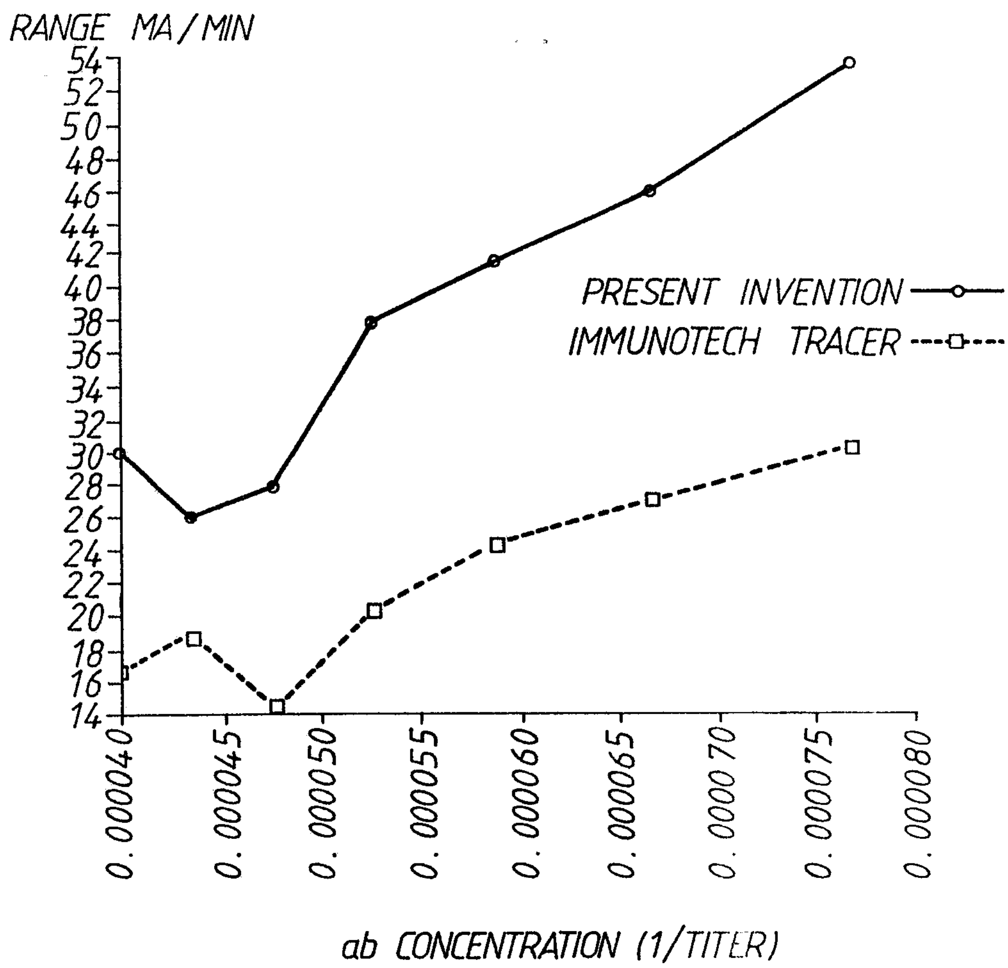


GRAPH III

European Patent Office

# EUROPEAN SEARCH REPORT

Application number

EP 88306243.2

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | DE - A1 - 3 215 294 (SCHERING AG) * Claim 1; pages 5,6,8,9 * -- | 1,3,5, 8,9,15 | C 07 K 17/00<br>G 01 N 33/74<br>G 01 N 33/535<br>C 12 N 9/00 |
| A | EP - A1 - 0 178 683 (NIHON MEDI-PHYSICS CO., LTD.) * Claims 1,3,6,21; pages 2,3 * -- | 1,5-9, 15 | |
| A | DE - B2 - 2 526 984 (THE RADIO-CHEMICAL CENTRE LTD.) * Claims 1,2; columns 3-6 * ---- | 1,2,8-15 | |
| | | | TECHNICAL FIELDS SEARCHED (Int. Cl.4) |
| | | | C 07 K 17/00<br>C 07 K 3/00<br>C 07 J 41/00<br>G 01 N 33/00<br>C 12 N 9/00<br>C 12 P 21/00 |

The present search report has been drawn up for all claims

| Place of search | Date of completion of the search | Examiner |
|---|---|---|
| VIENNA | 19-10-1988 | PETROUSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document